(19) Europäisches Patentamt
European Patent Office
Office européen des brevets

(11) **EP 2 511 703 B1**

(12) **EUROPÄISCHE PATENTSCHRIFT**

(45) Veröffentlichungstag und Bekanntmachung des Hinweises auf die Patenterteilung:
**01.04.2015 Patentblatt 2015/14**

(51) Int Cl.:
*G01N 33/00* (2006.01)       *G01N 33/46* (2006.01)
*G01N 27/62* (2006.01)

(21) Anmeldenummer: **12163211.1**

(22) Anmeldetag: **04.04.2012**

(54) **Verfahren zum Messen von emittierenden flüchtigen Stoffen aus Holzwerkstoffen und Vorrichtung zum Messen von emittierenden flüchtigen Stoffen aus Holzwerkstoffen**

Method for measuring emitting volatile materials from wooden materials and device for measuring volatile materials emitted from wooden materials

Procédé de mesure de matières volatiles émissives à base de matières dérivées du bois et dispositif de mesure de matières volatiles émissives à base de matières dérivées du bois

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.04.2011 DE 102011017280**

(43) Veröffentlichungstag der Anmeldung:
**17.10.2012 Patentblatt 2012/42**

(73) Patentinhaber:
• **Fagus-GreCon Greten GmbH & Co. KG**
**31061 Alfeld (DE)**
• **Georg-August-Universität Göttingen Stiftung Öffentlichen Rechts**
**37073 Göttingen (DE)**
• **Laser-Laboratorium Göttingen e.V. Abt. Photonische Sensorik**
**37077 Göttingen (DE)**

(72) Erfinder:
• **Wimmer, Rupert**
**5431 Kuchl (AT)**
• **Lenth, Christoph**
**37075 Göttingen (DE)**
• **Hasener, Jörg**
**31061 Alfeld (DE)**
• **Himmel, Sarah**
**37077 Göttingen (DE)**
• **Schumann, Achim**
**37574 Einbeck (DE)**

(74) Vertreter: **Jabbusch, Matthias**
**Jabbusch Siekmann & Wasiljeff**
**Patentanwälte**
**Roscherstrasse 12**
**30161 Hannover (DE)**

(56) Entgegenhaltungen:
**CA-A1- 2 009 062     DE-A1- 10 212 110**
**GB-A- 2 256 054     US-A- 5 071 771**

• **VAUTZ. W ET AL: "Detection of Emission from Material Surfaces using Ion Mobility Spectrometry", INTERNATIONAL JOURNAL FOR ION MOBILITY SPECTROMETRY, Bd. 7, Nr. 1, 1. Januar 2004 (2004-01-01), Seiten 25-29, XP002682163,**
• **TELGHEDER U ET AL: "Determination of volatile organic compounds by solid-phase microextraction-gas chromatography-differential mobility spectrometry", INTERNATIONAL JOURNAL FOR ION MOBILITY SPECTROMETRY NOVEMBER 2009 SPRINGER VERLAG DEU, Bd. 12, Nr. 4, November 2009 (2009-11), Seiten 123-130, XP002682164, DOI: DOI:10.1007/S12127-009-0032-Y**
• **HUA LI ET AL: "Separation of Ions from Volatile Organic Compounds Using High-Field Asymmetric Waveform Ion Mobility Spectrometry-Mass Spectrometer", CHINESE JOURNAL OF CHEMICAL PHYSICS IOP PUBLISHING LTD. UK, vol. 23, no. 2, April 2010 (2010-04), pages 125-132, ISSN: 1674-0068**

**Beschreibung**

Die Erfindung betrifft ein Verfahren zum Messen von emittierenden flüchtigen Stoffen aus Holzwerkstoffen, insbesondere von Formaldehyd. Des Weiteren betrifft die Erfindung eine Vorrichtung zum Messen von emittierenden flüchtigen Stoffen aus Holzwerkstoffen, insbesondere von Formaldehydverbindungen, insbesondere zur Durchführung des vorgenannten Verfahrens.

Holzwerkstoffe geben geringe Mengen an flüchtigen Kohlenwasserstoffen (VOC, Volatile Organic Compounds) aus Klebstoffen, aus dem Holz selbst bzw. aus Beschichtungsstoffen ab. Es gibt verschiede Definitionen zu VOC's. Gemäß der WHO werden VOC nach ihrem Siedepunkt und der daraus resultierenden Flüchtigkeit eingeteilt. Formaldehyd gehört danach zu den VVOC, da es einen Siedepunkt von -19°C hat.

| Beschreibung | Siedebereich |
|---|---|
| 1. Very Volatile Organic Compound (VVOC) | < 0 bis 50 ... 100°C |
| 2. Volatile Organic Compound (VOC) | 50...100 bis 240...260°C |
| 3. Semi Volatile Organic Compound (SVOC) | 240...260 bis 380...400°C |

Die in Deutschland geltende Richtlinie 1999/13/EG vom 11. März 1999 über die Begrenzung von Emissionen flüchtiger organischer Verbindungen definiert VOC als organische Verbindung, die bei 293,15 Kelvin einen Dampfdruck von 0,01 Kilopascal oder mehr hat oder unter den jeweiligen Verwendungsbedingungen eine entsprechende Flüchtigkeit aufweist.

Die nachträgliche Abgabe von Formaldehyd, wegen seiner mengenmäßigen Bedeutung meist gesondert von den VOC/VVOC- Emissionen betrachtet, zählt zu den wichtigen Qualitätskriterien bei Holzwerkstoffen. In Europa liegt der derzeitige gesetzliche Grenzwert für Formaldehydemissionen aus Holzwerkstoffen bei 0,1 ppm (Emissionsklasse E1). Da 2004 durch die Internationale Agentur für Krebsforschung (IARC) der Weltgesundheitsorganisation (WHO) Formaldehyd als "krebserregend" für den Menschen klassifiziert wurde, kam es in den letzten Jahren allerdings weltweit zu der Einführung von Standards unterhalb dieses Grenzwertes. Die Hauptquelle der Formaldehydemission aus Holzwerkstoffen ist das eingesetzte Bindemittel. Nach dem Pressvorgang verbleiben in den Holzwerkstoffen, auf Grund nicht vollständig vernetzter Klebstoffe, geringe Mengen an freiem Formaldehyd, die mit der Zeit an die Umgebung abgegeben werden. Diese Emissionsrate von Formaldehyd wird bisher nur durch die Wahl der Edukte gesteuert. Wird der Prozess gestört oder entsprechen die Edukte doch nicht den Erwartungen, kann dies nicht während des Prozesses selbst festgestellt werden, sondern nur bei der Endkontrolle. Dies führt zu einer Prozessunsicherheit und eventuell zu Produktionsausschuss. Zudem ist die nachträgliche Prüfung des Endproduktes sehr zeitaufwändig und wird bei den sinkenden Emissionspotentialen immer ungenauer. Gerade bei kontinuierlichen Produktionsprozessen von Holzwerkstoffen wie z. B. Spanplatten, mitteldichten Faserplatten (MDF) oder Oriented Strand Boards (OSB) ist somit eine kontinuierliche Prozesskontrolle bis hin zur Prozesssteuerung wünschenswert. Eine solche Prozesssteuerung kann aber bisher nicht durchgeführt werden, da eine entsprechende prozesstaugliche Vorrichtung zur Messung von flüchtigen Substanzen noch nicht entwickelt wurde.

Neben Formaldehyd emittiert Holz und somit auch Holzwerkstoffe in geringen Mengen weitere flüchtige Kohlenwasserstoffe (VOC) wie z. B. Terpene und organische Säuren (Essigsäure) (RISHOLM-SUNDMAN ET AL. 1998). Die Emissionsrate hängt von der Holzart, den Lagerungs-, Trocknungsbedingungen sowie den Produktionsfaktoren ab. Verschiedene Methoden zur Erfassung flüchtiger organischer Verbindungen wurden für Labormessungen bereits entwickelt. Bestimmungen zur Festlegung von Grenzwerten werden seit einiger Zeit diskutiert und erarbeitet.

Für die Erstprüfung und die regelmäßige Fremdüberwachung der Formaldehydemission des Produkts muss die "Kammer-Methode" nach EN 717-1 (1998) durchgeführt werden. Die Emissionsmessungen werden bis zur Erreichung der Ausgleichskonzentration bzw. bis maximal 28 Tage fortgesetzt. Hierbei werden die Formaldehydkonzentrationen in wässrigen Lösungen mit der Acetylaceton Methode photometrisch bestimmt. Die Formaldehydemission wird anschließend über das Prüfkammervolumen und die gemessene Formaldehydkonzentration berechnet. Die Formaldehydemission wird im Bezug zur umgebenden Luft in mg/m$^3$ bzw. ppm angegeben. Da die Kammermethode sehr zeitaufwendig ist, sind sog. abgeleitete Prüfmethoden für die laufende Produktionsüberwachung im Sinne einer Endkontrolle entwickelt worden.

Die Gasanalyse-Methode (EN 717-2 1994) ist ein Schnellmessverfahren, welches für die werkseigene Überwachung von beschichteten Holzwerkstoffen zugelassen ist. Hierbei handelt es sich, wie bei der beschriebenen Kammer-Methode, um eine Emissionsmessung, auch die nasschemische Analyse ist die gleiche. Lediglich die Berechnung der Emissionswerte ist eine andere. Mit dieser Methode, auch wenn sie als Schnellmethode bezeichnet wird, dauert es min. 6 Stunden bis die Formaldehydemission eines Holzwerkstoffes bestimmt ist.

Die Perforator-Methode zur Messung des Formaldehydgehaltes (EN 120 1992) ist das zurzeit gängige und eines zur Produktionsüberwachung vorgeschriebene Prüfverfahren in Europa für unbeschichtete Holzwerkstoffe. Dieses gilt ebenfalls als Schnellmessverfahren. Aber auch dieses Verfahren ist in Durchführung und Auswertung anspruchsvoll; Ergebnisse werden erst nach mindestens 5 Stunden geliefert. Ein endgültiges Ergebnis steht erst nach 24 Stunden zur Verfügung, d. h. erst in der nächsten Schicht bzw. am nächsten Tag.

Der sichere Nachweis von Formaldehydgehalten < 4 mg HCHO/100g atro Probe und dementsprechend niedrigen Emissionsraten, wie sie die neuen Grenzwerte z.B. nach dem kalifornischen Air Resources Board ("CARB"), IKEA (IOS-MAT-0003) oder nach japanischen Standards (F**** nach JIS oder JAS) fordern, stellt eine Herausforderung an das verwendete Analysenverfahren dar. Es hat sich gezeigt, dass die mit der Perforator-Methode erreichbare Nachweisgrenze zu hoch liegt. Zudem korreliert die mit der Perforator-Methode ermittelte Formaldehyd-Konzentration bei derart niedrigen Messwerten nicht mehr hinreichend mit der Emissionsrate von Formaldehyd aus dem Werkstoff. Nach RISHOLM-SUNDMAN ET AL. (2007) erklärt sich die ungenügende Korrelation zwischen den Perforatorwerten und den Emissionswerten dadurch, dass die Formaldehydemission von Holzwerkstoffeigenschaften wie z.B. von der Rohdichte, Porosität und Rohstoffe abhängt. Die Perforator-Methode ist somit, angesichts der niedrigen Grenzwertvorgaben, nicht mehr als geeignete Methode anzusehen, um die Formaldehydemissionsrate bei Holzwerkstoffen zu bestimmen.

Im Gegensatz dazu sind mit der Gasanalyse- Methode sehr gute Korrelationen mit den Referenzmethoden nach EN- und ASTM Standard erreichbar (MARUTZKY 2010). Allerdings macht der hohe Zeitaufwand der zur Produktionsüberwachung beschriebenen Methoden es für die Holzwerkstoffindustrie schwierig, eine effektive Prozesskontrolle zu realisieren. Die erhaltenen Daten können durch die hohe Zeitverzögerung nur bedingt zur Prozesssteuerung beitragen. Hierfür besteht die Notwendigkeit der Verfügbarkeit von Echtzeit-Messverfahren/-vorrichtungen, definiert durch ausreichend kurze Messintervallzyklen, so dass in den Prozess eingegriffen werden kann.

Zur Bestimmung der Emission von flüchtigen organischen Verbindungen (VOC) aus Materialien und Produkten findet das Prüfkammerverfahren nach EN 13419-1 (2003) und nach ISO 16000-9 (2008) (früher: EN 13419-1) Anwendung. Die Emissionsprüfung findet in einer Prüfkammer aus Glas statt. Die Luftprobennahme kann über zwei verschiedenen Adsorptionsmedien vorgenommen werden. Zum einen über das international am weitesten verbreitete Tenax TA (Edelstahlrohr zur Adsorption), welches anschließend thermodesorbiert wird damit die adsorbierten Stoffe freigesetzt werden. Zum anderen wird, vor allem auch in Deutschland, Aktivkohle in Glasrohren zur Adsorption verwendet. Die adsorbierten Stoffe werden anschließend von der Aktivkohle mit Hilfe von Schwefelkohlenstoff gelöst. (Lösungsmitteldesorption) Die bei beiden Methoden freiwerdenden Verbindungen werden gaschromatographisch getrennt und in einem Massenspektrometer identifiziert und quantifiziert. Diese Referenzmethode dauert 28 Tage bis die endgültigen Ergebnisse vorliegen.

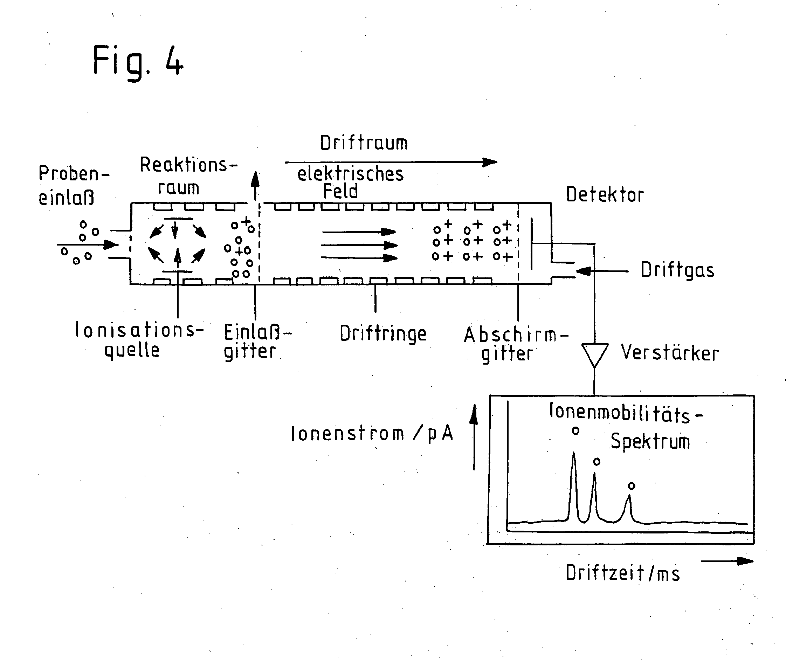

Die klassische Ionenmobilitätsspektrometrie ist ein analytisches Detektionsverfahren, bei dem unter Normaldruck erzeugte Ionen in einem elektrischen Feld gegen die Strömungsrichtung eines Trägergases driften. Ionen unterschiedlicher Masse und/oder Struktur erreichen hierbei unterschiedliche Driftgeschwindigkeiten und treffen daher zeitlich versetzt auf den Detektor, siehe Figur 4.

Diese Methode eignet sich zum Nachweis von allen Stoffen, die in Luft oder Stickstoff ionisiert werden können. Die häufigste verwendete Ionisationsmethode ist die chemische Ionisation mit Hilfe eines $\beta$ - Strahlers (z. B. $^{63}$Ni). Wird Luft als Trägergas verwendet, entstehen zunächst sogenannte Reaktions- oder Primärionen sowohl positiver als auch negativer Polarität. Liegen gleichzeitig Probenmoleküle im Reaktionsraum vor, die ein größeres Bestreben haben, diese Ladungen zu übernehmen, so findet eine Ladungsübertragung von den Reaktions-Ionen zu den neutralen Probenmolekülen statt. Die auf diese Art entstandenen Produkt- oder Sekundärionen gelangen schließlich durch das Einlassgitter in den Driftraum, in welchem sie sich gegen die Strömungsrichtung eines neutralen Driftgases und unter dem Einfluss des elektrischen Feldes in Richtung des Detektors bewegen und schließlich als zeitabhängiges Stromsignal registriert werden.

Das Stromsignal (Peak), welches durch die Reaktions-Ionen hervorgerufen wird, ist im Spektrum weitestgehend immer an der gleichen Stelle (Driftzeit) zu finden. Produkt-Ionen, welche zufällig gleiche Driftzeiten aufweisen können durch eine Peaküberlagerung nicht ausgewertet werden; dies trifft insbesondere bei der Detektion von Formaldehyd auf.

Trotzdem wurde IMS in der Vergangenheit zur Messung von Formaldehyd und VOC in der Raumluft verwendet. Um die driftzeitmäßige Trennung des Reaktionsionen-Peaks vom Formaldehydpeak zu erreichen kann dem Analysegas Ammoniak zugesetzt werden (Leonhardt J., Leonhardt M., und Bensch H. 2003). Aus dem Patent von Leonhardt et. al. (2003) geht allerdings nicht hervor, ob Sie sicher Formaldehyd nachweisen. Die Autoren lassen offen nach welchem Ladungsübertragungsmechanismus die Detektion erfolgen soll.

Auch die Art der Kalibrierung des Systems wird nicht beschrieben. Zusammenfassend lässt sich sagen, dass die größten Nachteile der klassischen IMS die mangelnde Selektivität und der eingeschränkte Dynamikbereich sind.

Die NIR-Spektroskopie in Kombination mit multivariater Datenanalyse (MVA) wurde in den letzten Jahren als Methode zur Kontrolle von Materialeigenschaften wie Holzfeuchte, Spangröße- und Form sowie Holzqualität und auch der Vorhersage von Platteneigenschaften in der holzverarbeitenden Industrie zunehmend eingesetzt. SO et al. (2004) hat hierzu eine Rezension veröffentlicht. Im Zuge der Anstrengungen der letzten Jahre, Systeme und Verfahren zur online-Formaldehydüberwachung in der Produktion zu entwickeln, stand daher die Nahinfrarotspektroskope (NIR-Spektroskopie) im Fokus der Aufmerksamkeit (ENGSTRÖM 2008, ANDRE et al. 2009, SOBEC 2009, KIM 2010). Von Holzspänen oder Plattenoberflächen aufgenommene NIR-Spektren werden hierbei mit Daten aus klassischen Referenzmessverfahren korreliert. So können die Formaldehydemissionen von Holzwerkstoffen durch die Aufnahme von NIR-Spektren über die bestehende Kalibrierung mit statistischen Verfahren geschätzt werden. Allerdings kann mittels NIR-spektroskopischer Verfahren die Konzentration oder Emissionsrate von Formaldehyd nicht direkt gemessen werden.

Die Nahinfrarotspektroskopie wird in ihrer Leistungsfähigkeit durch große spektrale Interferenzen und hohe Matrixeinflüsse limitiert. Sie eignet sich daher zwar zur Schätzung der Konzentrationen von Hauptkomponenten in Gemischen aber nicht zur Spurenanalytik. Formaldehyd in Holzwerkstoffen ist allerdings ein Spurenbestandteil, der nur schwer zu detektieren ist. Es ist daher wahrscheinlich, dass die in der Literatur erwähnten Verfahren auf Basis der Nahinfrarotspektroskopie nur einer indirekten Korrelation zu Grunde liegen. D. h. die Veränderungen in den Spektren werden nicht durch unterschiedliche Formaldehydkonzentrationen verursacht, sondern durch andere Faktoren, wie der Klebstoffmenge und der Klebstoffzusammensetzung oder chemischen Unterschieden zwischen Holzsorten, welche wiederum mit der Formaldehydkonzentration korrelieren. Dieser Sachverhalt führt dazu, dass nach Einschätzung des Laser-Laboratorium Göttingen e.V. die Zuverlässigkeit NIR-basierter Messverfahren kritisch zu betrachten ist. Die Tatsache, dass sich die NIR-Technologie bezüglich der Formaldehydbestimmung in keiner Weise in der Prozessanalytik hat durchsetzen können, bestätigt die Bedenken.

VAUTZ. W ET AL: "Detection of Emission from Material Surfaces using Ion Mobility Spectrometry", INTERNATIONAL JOURNAL FOR ION MOBILITY SPECTROMETRY, Bd. 7, Nr. 1, 1. Januar 2004 (2004-01-01), Seiten 25-29, XP002682163, offenbart ein Verfahren zum Messen von emittierenden flüchtigen Stoffen aus Holzwerkstoffen, insbesondere von Formaldehyd, dass von einem Holzwerkstoff einige Partikel als Messproben entnommen werden und diese Ionenmobilitätsspektrometrisch untersucht werden.

Der Erfindung liegt die Aufgabe zu Grunde, ein Verfahren der eingangs genannten Gattung aufzuzeigen, mit der ein Messen von Formaldehydemissionen und anderen flüchtigen Verbindungen in kurzer Zeit ermöglicht ist, so dass sowohl eine Regelung in einer Produktion von Holzwerkstoffen als auch eine fortlaufende Überwachung dieser Produktion ermöglicht ist. Weiterhin soll eine Vorrichtung vorzugsweise zum Durchführen des vorgenannten Verfahrens aufgezeigt werden.

Verfahrensseitig ist diese Aufgabe erfindungsgemäß ein Verfahren entsprechend dem Anspruch 1 gelöst.

Für das erfindungsgemäße Verfahren werden mehrere Proben aus einer zu prüfenden z. B. Holzwerkstoff-Platte entnommen. Die Proben können beispielsweise Bohr- oder Sägespäne, Bohrkerne oder Probekörper sein, es können aber auch direkte Gasproben verwendet werden.

Diese Partikel werden einer Ionenmobilitätsspektrometrie zugeführt, mit der eine schnelle Messung auf emittierende flüchtige Stoffe möglich ist. Die Messdauer beträgt bis zur Messwertausgabe lediglich einige Sekunden bis maximal wenige Minuten. Auf den Einsatz gesundheitsgefährdender Gefahrenstoffe, wie z. B. Toluol bei der Perforatormethode oder Schwefelkohlenstoff wie bei der Extraktion von flüchtigen Stoffen von Adsorbaten, kann vorteilhaft verzichtet werden.

Mit der Ionenmobilitätsspektrometrie gelingt dann ein sehr genauer Nachweis auch geringer Mengen an flüchtigen Stoffen.

Vorzugsweise wird das erfindungsgemäße Verfahren in Echtzeit während der Herstellung des Holzwerkstoffes durchgeführt. Hierbei sind die Zeitintervalle zwischen den Messungen ausreichend kurz definiert, um Eingriffe in den Prozess zwecks Qualitätsverbesserung und Ausschussverringerung zu ermöglichen. Alternativ kann auch ein Laborversuch aufgebaut werden.

Nach einer Weiterbildung der Erfindung ist vorgesehen, dass die Partikel nach dem Entnehmen erhitzt werden. Dabei kann die Probe in einem geschlossenen Gefäß erhitzt werden. Durch das Erhitzen gasen die zu analysierenden Stoffe aus. Die so gewonnene gasförmige Probe kann anschließend mittels Ionenmobilitätsspektrometrie untersucht werden, dass für die Ionenmobilitätsspektrometrie ein FAIMS System (Field Asymmetric Ion Mobility Spectrometer) verwendet wird. Alternativ kann von vorne herein von einer abgesaugten Gasprobe von Partikeln oder direkt von der Holzwerkstoff-Platte ausgegangen werden.

Die beim Erhitzen freigesetzten Gase können vorzugsweise durch eine Adsorptionseinheit, beispielsweise ein Adsorptionsröhrchen geleitet werden, wodurch sie zunächst gesammelt werden (Anreicherung). Zur erneuten Freisetzung dieser Gase wird der Gasfluss gestoppt, das Röhrchen kurzzeitig mit einer Ventilkombination beidseitig geschlossen und das Adsorptionsmedium möglichst schnell erhitzt. Durch erneutes Öffnen des Röhrchens mit Hilfe der Ventilkombination und Umkehrung des Gasflusses werden die freigesetzten Gase auf die gaschromatographische Säule geleitet und voneinander getrennt. Die auf diese Art getrennten Gaskomponenten werden anschließend mit Hilfe der Ionenmobilitätsspektrometrie detektiert und identifiziert, dass die Anlage zur Ionenmobilitätsspektrometrie eine FAIMS-Anlage ist.

Im Rahmen der Anreicherung werden Formaldehyd und andere emittierte Substanzen durch Reaktion mit zugesetzten Chemikalien derivatisiert , was die anschließende Bestimmung erleichteret bzw. sicherer macht. Für Aldehyde bekannte Derivatisierungsverfahren sind die Reaktion mit Hydrazinen zu Hydrazonen, z. B. unter Verwendung von DNPH, DNSH, PFPH oder TCPH und die Reaktion mit Aminen zu Oximen unter Verwendung von Benzylhydroxylamin, Methoxyamin oder PFBHA. Als Beispiele für beide Reaktionstypen sind die DNPH-Methode (ISO 16000-3) und die von Martos und Pawliszyn (P. A. Martos, J. Pawliszyn, Anal. Chem. 1998, 70, 2311-2320) beschriebene Umsetzung von Aldeyhden mit PFBHA zu Oximen unter Verwendung von Solid Phase Micro Extraction zu nennen. Ein weiteres Derivatisierungsverfahren stellt die 2-HMP-Methode (VDI-Richtlinie 3862 Blatt 5, 2008) dar.

Es kann vorgesehen sein, dass die für das Verfahren verwendete Messvorrichtung mit Standardproben kalibriert wird. Dadurch wird die Genauigkeit des erfindungsgemäßen Verfahrens erhöht.

Alternativ kann vorgesehen sein, dass die beim Erhitzen frei gesetzten Gase unmittelbar in eine GC-Säule, vorzugsweise in einen Schnell-Gaschromatografen, eingeleitet werden.

Die Selektivität der Ionenmobilitätsspektrometrie wird maßgeblich durch eine derartige vorgeschaltete GC-Säule verbessert. Mit der GC-Säule wird das gasförmige Stoffgemisch in die einzelnen Substanzen getrennt. Da jeder Stoff eine definierte Zeit, die Retentionszeit, braucht, um die Säule zu passieren, ist bekannt, wann z. B. Formaldehyd aus dem GC in die Ionenmobilitätsspektrometrie gelangt. Es können durch eine Ventilsteuerung, die nicht zu analysierenden Stoffe bei Bedarf ausgeschleust werden. So kann die Analysenzeit verkürzt werden, wenn man mit zwei Säulen im Gaschromatografen arbeitet. Während aus der einen Säule die schwer flüchtigen Bestandteile ausgeschleust werden, ist die zweite Säule bereits mit der nächsten Probe beaufschlagt worden. Auch kann so bei Bedarf verhindert werden, dass das Ionenmobilitätsspektrometrie-System durch schwer flüchtige Bestandteile verschmutzt ein störendes Dauersignal ausgibt. Der in dieser Erfindung eingesetzte GC braucht keine andere Gasversorgung als gereinigte Luft, die durch die Säule gepumpt wird.

Für die Ionenmobilitätsspektrometrie ein FAIMS System (**F**ield **A**symmetric **I**on **M**obility **S**pectrometer) verwendet wird.

Diese IMS-Ausbildungen werden als Mikro-DMS (**D**ifferential-Ionen-**M**obilitä**t**sspektroskopie) oder auch **FAIMS** bezeichnet. Bei der FAIMS wird das elektrische Feld nicht mit Hilfe hintereinander angeordneter Ringe aufgebaut, sondern durch zwei Kondensatorplatten, zwischen die das Trägergas (gereinigte Luft oder Stickstoff) hindurch strömt.

Die technische Anordnung ermöglicht im Gegensatz zur klassischen IMS eine simultane Erfassung negativer und positiver Ionen. Zwischen den Plattenelektroden wird ein asymmetrisches Wechselfeld mit einer Spannung von mehreren kV angelegt, was die Messung einiger VOC, insbesondere Formaldehyd, erst ermöglicht. Das resultierende elektrische Wechselfeld zwingt die Ionen auf dem Weg durch die Driftröhre zu periodischen Richtungswechseln gleicher Frequenz ("Zickzackkurs"). Je nach Ionenmobilität driften die einzelnen Spezies entweder bis zum Detektorausgang oder werden an einer der beiden Elektroden entladen. Durch das Anlegen einer variablen Gleichspannung kann diese Ionen-Bewegung so gesteuert werden, dass je nach Spannungspegel eine andere Spezies bis zum Detektor gelangt ("tunable Ionfilter"). Hierbei ist zu beachten, dass die Ionenmobilität nur bei geringen elektrischen Feldstärken unabhängig von eben dieser Feldstärke ist.

Durch die Einführung der asymmetrischen Beschleunigungsfelder (Variation der Hochspannung (DV, Dispersion Voltage)) wurde die Selektivität und Empfindlichkeit der Analyse erheblich verbessert und der apparative Aufwand erheblich verringert. Die Verbesserung der Selektivität gegenüber IMS ist eine direkte Folge der Verwendung sehr hoher elektrischer Feldstärken, da sich bei Einwirkung dieser starken elektrischen Felder die Ionenmobilität auf substanzspezifische Weise mit der Feldstärke ändert. Dadurch lassen sich FAIMS-Geräte preiswert und vor allem miniaturisiert herstellen, da sie keine Schaltgitter zum Probeneinlass und Abschirmgitter vor dem Detektor benötigen. Durch die Variation der Hochspannung ermöglicht die FAIMS-Technologie, im Gegensatz zur klassischen IMS, die Trennung der Reaktions-Ionen und der Produkt-Ionen, so dass es nicht mehr zu einer Überlagerung der Ionen-Peaks bei der Formaldehyddetektion kommt. Des Weiteren ist die Geschwindigkeit der Trennung und Detektion sehr hoch und kann weniger als 1 Sekunde betragen (MILLER et al. 2001).

Das FAIMS gibt ein Dispersionsplot und die gemessen Spektren des Detektorsignals über die Kompensationsspannung aus (siehe Anlage). Über die im System hinterlegte Kalibrationsfunktion wird die aus der Probe emittierte Stoffmenge aus dem Spektrums berechnet und in ppm oder ppb angezeigt.

Für quantitative Analysen mit der FAIMS-Technik ist eine Kalibrierung des Systems erforderlich. Diese erfolgt durch das Permeationsverfahren nach ASTM Designation D3609-96. Permeationsröhrchen werden mit einer Reinform der zu analysierenden Substanz gefüllt. Für die Kalibrierung des FAIMS auf Formaldehyd werden Röhrchen mit Paraformaldehyd gefüllt, welche auf dem Markt erhältlich sind. Diese Röhrchen werden in eine kleine Kammer (Permeationskammer) eingeführt. Hier gewährleisten eine konstante Temperatur von 90°C eine Depolymerisation des Paraformaldehyds zu gasförmigem Formaldehyd und sowie ein gleich bleibender Gasfluss den konstanten Massendurchfluss des zu analysierenden Stoffes. Über den Masseverlust der Röhrchen in der Permeationskammer kann die Permeationsrate gravimetrisch bestimmt werden.

$$C_{ppmv} = \frac{R \bullet V_m}{MG \bullet (F1 + F2)}$$

$$C_m = C_{ppmv} \bullet MG \bullet \left(\frac{1000}{24{,}47}\right) \bullet \left(\frac{P}{101{,}3}\right) \bullet \left(\frac{298{,}15}{(T + 273{,}15)}\right)$$

| | |
|---|---|
| R | gravimetrisch ermittelte Permeationsrate [$\mu$g/min] |
| $V_m$ | Molvolumen [l/min], bei 25°C und 101,3 kPa |
| MG | molare Masse [g/mol] |
| F1 | Verdünnungsflussrate [l/min] |
| F2 | Flussrate durch die Permeationskammer [l/min] |
| P | Atmosphärendruck [kPa] |
| T | Temperatur [°C] |
| $C_{ppmv}$ | Konzentration [ppm] |

Die Formaldehydkonzentration ($C_{ppmv}$) kann nach Ermittlung der Permeationsrate nach der oben stehenden Formel (Formel [1]) berechnet werden.

Die eingestellte Formaldehydkonzentration, welche aus der Permeationskammer austritt, kann dann, ggf. nach Derivatisierung, mit FAIMS oder GC-FAIMS gemessen werden. Somit wird eine Kalibrationsfunktion erstellt und in der Software des FAIMS Systems hinterlegt. Dies kann nach demselben Prinzip für verschiedene Stoffe erfolgen und sollte nach in definierten Zyklen wiederholt werden.

Mit dem erfindungsgemäßen Verfahren gewonnene Erkenntnisse können unmittelbar auf den Herstellungsprozess der Holzwerkstoffe zurückgeführt werden. Dazu werden die ionenmobililtätsspektrometrische Untersuchung der Partikel mit einer Steuerung für eine Produktionsstraße für Holzwerkstoffe verschaltet. Gemessene Eigenschaften der Holzwerkstoffe können dann unmittelbar in den Herstellungsprozess zurückgeführt werden. Die Messung der Holzwerkstoffe erfolgt in Echtzeit, ihre Ergebnisse können dann auch in Echtzeit unmittelbar in den Produktionsprozess eingefügt werden.

Die vorrichtungsseitige Lösung der oben genannten Aufgabe ist dadurch gekennzeichnet, dass die Vorrichtung zumindest eine Einrichtung zur Probenentnahme von Partikeln der Holzwerkstoffe sowie wenigstens eine Anlage für die Ionenmobilitätsspektrometrie aufweist.

Um die oben ausgeführte Ionenmobilitätsspektrometrie auszuführen, sind den Holzwerkstoffen einige Proben zu entnehmen. Diese Proben sind insbesondere als Späne ausgebildet, wie sie beispielsweise an Sägen, Bohr-Schleif- bzw. Hobelmaschinen auftreten. Von diesen Werkzeugen werden Späne häufig in Leitungen abgeführt, beispielsweise abgesaugt.

Nach einer Weiterbildung der erfindungsgemäßen Vorrichtung ist daher vorgesehen, dass die Einrichtung zur Probenentnahme eine Abführleitung für Säge- und/oder andere Späne der Holzwerkstoffe zugeordnet ist, wobei wenigstens ein in die Abführleitung eingesetztes Ausschleusungselement für die Späne vorgesehen ist. Das Ausschleusungselement kann Späne zunächst hindurchlassen, um sie einer Entsorgung oder Weiterverwertung zuzuführen. Wenn allerdings für die erfindungsgemäße Messung Partikel benötigt werden, wird eine Stellung des Ausschleusungselementes verändert, so dass Späne zu einer Ionenmobilitätsspektrometrie geführt werden können. Das Ausschleusungselement kann einen mechanischen oder auch einen maschinellen Antrieb aufweisen.

Es kann vorgesehen sein, dass in eine vom Ausschleusungselement zur Anlage für die Ionenmobilitätsspektrometrie verlaufende Abzweigungsleitung für die Späne ein Zyklonbehälter und in Späneführrichtung nachfolgend eine Zellen-radschleuse eingesetzt ist. In einem Zyklonbehälter kann eine Spänemenge gesammelt werden, über die Zellenradschleuse kann sie aus einem druckbelasteten System ohne Druckverlust ausgeschleust werden.

Die Anlage zur Ionenmobilitätsspektrometrie ist wie im Verfahren bevorzugt angegeben eine FAIMS-Anlage. Die FAIMS-Anlage kann zumindest ein Multikanalhochfeldionenfilter-Spektrometer bevorzugt im Druckbereich von 50 mbar bis 5000 mbar sein.

Das für die vorliegende Vorrichtung verwendete Gerät der Fa. Owlstone, Modell Lonestar (WO 2006/046077; US 2008/054174; IL 181140; US 2008/017790; US 2010/213364; WO 2008/094299; WO 2009/136190) ist ebenfalls eine Variante des FAIMS, unterscheidet sich jedoch durch Feldstärken von bis zu 80 kV, einem Arbeitsdruck von 50 bis 5000 mbar und einer Vielzahl von miniaturisierten Kondensatorplatten (anstatt zwei Platten) von der DMS und kann als Multikanalhochfeldionenfilter-Spektrometer (MHIS) bezeichnet werden.

Derartige Geräte lassen sich in sehr kleiner Bauweise und preiswert herstellen, da sie zudem keine Schaltgitter zum Probeneinlass und Abschirmgitter vor dem Detektor benötigen. Durch die Variation der Hochspannung ermöglicht Multikanalhochfeldionenfilter-Spektroskopie, im Gegensatz zur klassischen IMS, die Trennung der Reaktions-Ionen und der Produkt-Ionen, so dass es nicht mehr zu einer Überlagerung der Ionen-Peaks bei der Formaldehyddetektion kommt. Des Weiteren ist die Geschwindigkeit der Trennung und Detektion sehr hoch und kann weniger als 1 Sekunde betragen (Miller et al. 2001).

Vorrichtungsseitig kann noch vorgesehen sein, dass die Anlage zur Ionenmobilitätsspektrometrie mit zumindest einer Steuerung für eine Produktionsstraße für Holzwerkstoffe verschaltet ist. Dadurch ergibt sich eine Online-Überwachung der Produktion, indem das erfindungsgemäße Verfahren direkt in der oder an der Produktionsstraße eingesetzt wird.

Die vom FAIMS System ausgegebenen Messwerte werden in die Prozessdatenkontrolle integriert und im Prozessleitstand sowie am Gerät auf einem Bildschirm angezeigt. Dies ermöglicht eine kontinuierliche Überwachung der produzierten Werkstoffes im Gegensatz zu den jetzigen Stichprobenkontrollen, wobei meist nur eine Platte pro Schicht getestet wird.

Aufgrund der kontinuierlichen Überwachung des Endproduktes können Änderungen in der Prozesssteuerung durch eine Person oder sogar automatisiert vorgenommen werden, um das Emissionsverhalten der Holzwerkstoffe den produktspezifischen Vorgaben anzupassen. Die Formaldehydemission eines Holzwerkstoffes kann unter anderem durch Veränderungen der Beleimungsrezepte (Leimtyp, Formaldehyd-fängerzugabe, etc.) sowie einiger Prozessparameter (Presszeit, Spanfeuchte, Presstemperatur, etc.) kurzfristig angepasst werden. Eine Veränderung des Spangemisches ist ebenfalls möglich. Dies führt aber erst mittelfristig zu Emissionsänderungen des Endproduktes, da die Rohstoffmischung am Anfang der Prozesskette steht.

Ausführungsbeispiele der Erfindung, aus denen sich weitere erfinderische Merkmale ergeben, sind in der Zeichnung dargestellt. Es zeigen:

Fig. 1: eine schematische Darstellung der erfindungsgemäßen Vorrichtung nach einem ersten Ausführungsbeispiel

Fig. 2 & 3: eine Draufsicht und eine perspektivische Ansicht eines Ausschleusungselementes der Vorrichtung gemäß Fig. 1,

Fig.4: ein schematischer Aufbau und ein Messbetrieb eines Ionenmobilitätsspektrometers nach dem Stand der Technik,

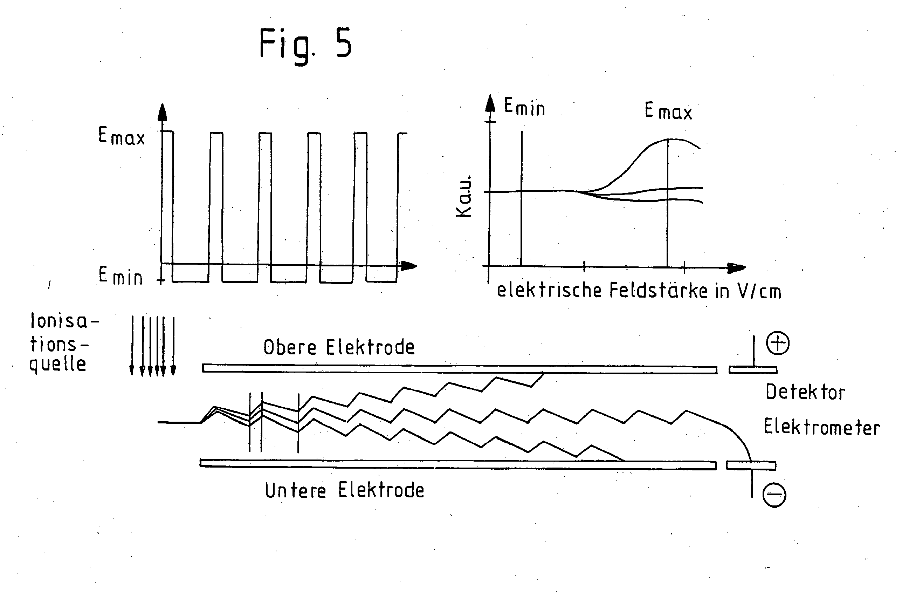

Fig. 5: einen schematischen Aufbau sowie das Messprinzip eines **F**ield **A**symmetric **I**on **M**obility **S**pectrometers (FAIMS), und

Fig. 6: eine schematische Darstellung der erfindungsgemäßen Vorrichtung nach einem zweiten Ausführungsbeispiel.

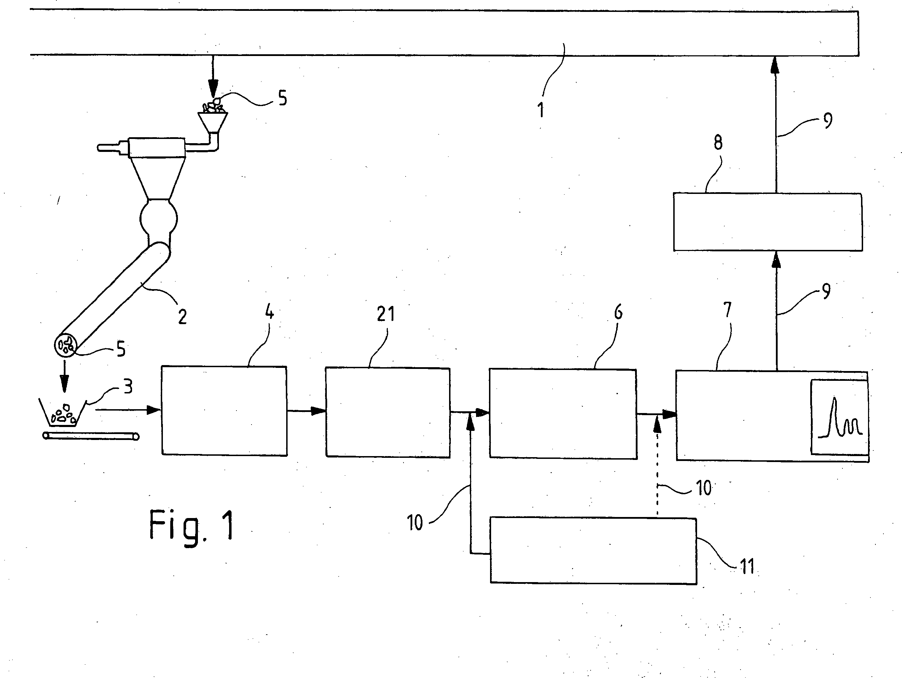

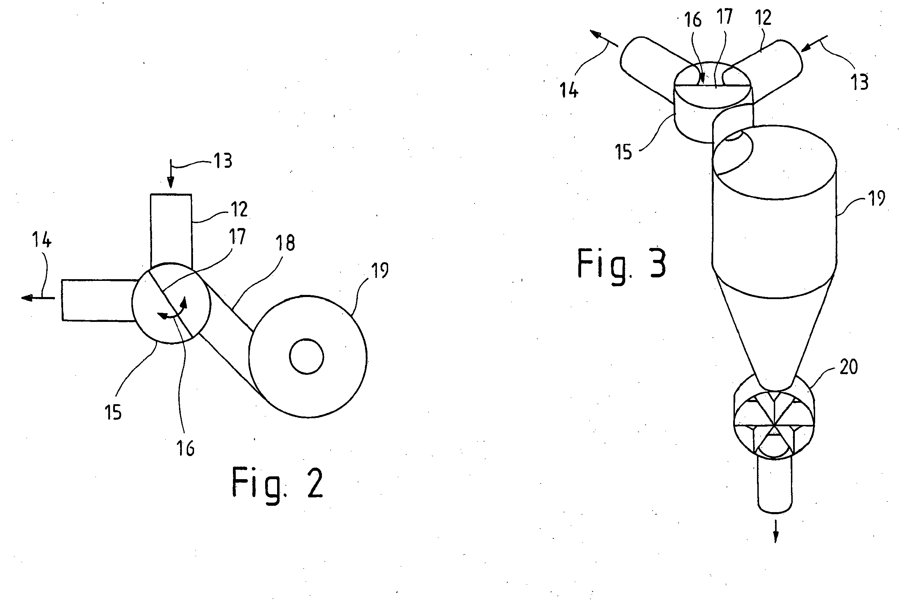

Im oberen Bereich der Figur 1 ist schematisch eine Produktionsstraße 1 dargestellt. Dieser sind das erfindungsgemäße Verfahren sowie die erfindungsgemäße Vorrichtung zugeordnet. Die Vorrichtung umfasst eine pneumatische Förderstrecke (Fig 2 & 3) 2 aufweisende Probenzufuhr 2, mit der Partikel 5 noch über einen weiteren Behälter 3 einem Ofen 4 zugeführt werden. In diesem Ofen 4 werden die als Messproben entnommenen Partikel 5 erhitzt.

Beim Erhitzen frei werdende Gase werden anschließend in eine GC- Säule 6 eingeführt. Dieser GC-Säule 6 ist eine Adsorptionseinheit 21 vorgeschaltet. Aus der gaschromatografischen Säule 6 austretende Stoffe werden dann einer FAIMS-Anlage 7 zugeführt. Hier erfolgt eine Messung der einzelnen Bestandteile der Gase.

Wird bei dieser Messung ein zu hoher Anteil von z. B. Formaldehyd festgestellt, erfolgt ausgehend von der FAIMS-Anlage 7 ein Eingriff auf die Prozesssteuerung 8 für die Produktionsstraße 1. Der Eingriff ist durch Pfeile 9 verdeutlicht.

Der GC- Säule 6 kann entlang der Pfeile 10 noch ein Referenzstandard 11 zum Zwecke der Kalibration zugeführt werden.

Fig. 2 zeigt in Draufsicht eine Abführleitung 12 für Säge- und andere Späne der Holzwerkstoffe. In diese Abführleitung 12 werden die Späne entlang des Pfeils 13 eingeführt, entlang Pfeil 14 erfolgt eine weitere Führung. Auf dem Weg zwischen Pfeil 13 und Pfeil 14 passieren die Späne in der Abführleitung 12 ein Ausschleusungselement 15. Dieses Ausschleusungselement 15 ist als Trommel ausgebildet, es nimmt in seinem Inneren eine entlang eines Doppelpfeils 16 bewegbare Trennwand 17 auf. In Fig. 3 ist die Stellung der Trennwand 17 gezeigt, in der die Späne durch die Abführleitung 12 bis zum Pfeil 14 geführt werden können. Fig. 2 zeigt dagegen die Stellung der Trennwand 17, bei der Späne aus der Abführleitung 12 abgezweigt werden und über eine Abzweigungsleitung 18 in einen Zyklonbehälter 19 eingeführt werden. In diesem können Späne gesammelt werden. Am unteren Ausgang des Zyklonbehälters 19 ist eine Zellenradschleuse 20 angeordnet. Mit dieser können dem Zyklonbehälter 19 definierte Mengen von Spänen für eine Überprüfung als Partikel 5 entnommen werden.

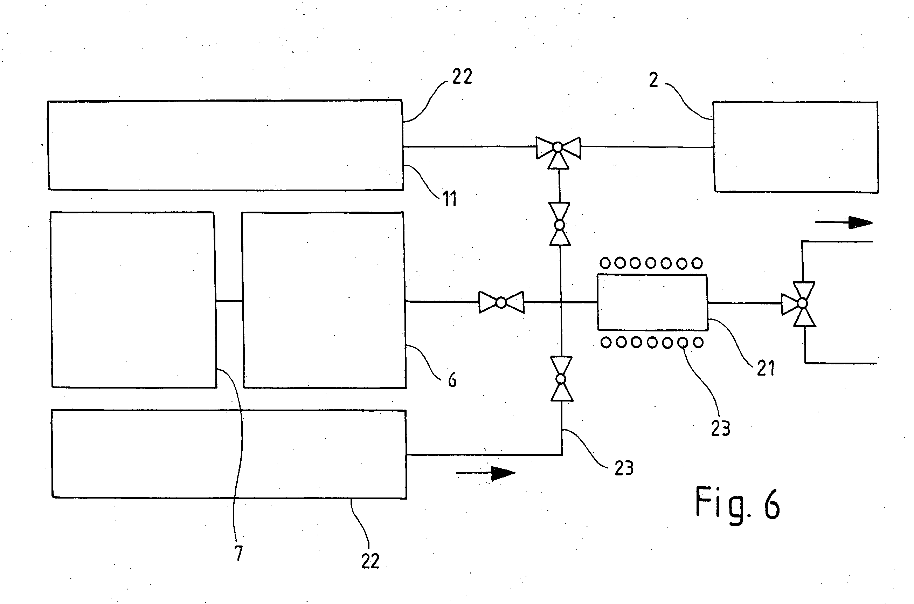

Bei dem Ausführungsbeispiel in Fig. 6 ist der Adsorptionseinheit 21 ein Standardgasgenerator 11 zugeordnet. Von diesem kann ein Formaldehyd-Kalibriergas zur Verfügung gestellt werden. Der Adsorptionseinheit 21 ist ein Heizsystem 23 zugeordnet.

Fig. 6 zeigt, dass von einem zweiten Standardgasgenerator 22 ein Derivatisierungsmittel bereitgestellt werden kann und über eine Leitung 23 in die Adsorptionseinheit 21 aufgegeben wird.

**Patentansprüche**

1. Verfahren zum Messen von emittierenden flüchtigen Stoffen aus Holzwerkstoffen, insbesondere von Formaldehyd, bei dem von einem Holzwerkstoff einige Partikel (5) als Messproben entnommen werden und diese ionenmobilitätsspektrometrisch untersucht werden, wobei für die Ionenmobilitätsspektroskopie eine FAIMS (7) (Field Asymmetric Waveform Ion Mobility Spectrometry) verwendet wird,
**dadurch gekennzeichnet,**
dass die Proben (5) nach dem Entnehmen erhitzt oder bei Raumtemperatur analysiert werden, bei einem Erhitzen oder bei Raumtemperatur frei gesetzte Gase durch eine Adsorptionseinheit (Anreicherungseinheit) mit angeschlossener thermischer Desorptionseinheit geführt werden und anschließend in eine gaschromatografische Säule (6) eingeleitet werden und dass die freigesetzten Gase insbesondere in der Adsorptionseinheit eine Derivatisierung durch Reaktion mit zugesetzten Chemikalien erfahren.

2. Verfahren nach Anspruch 1, **dadurch gekennzeichnet, dass** es in Echtzeit während der Herstellung des Holzwerkstoffes durchgeführt wird.

3. Verfahren nach Anspruch 1 oder 2, **dadurch gekennzeichnet, dass** die als Messproben entnommenen Partikel (5) in Form von Bohr-, Hobel- , Schleif- oder Sägespänen, Bohrkernen oder Probekörpern oder direkte gasförmige Proben von Holz und Holzwerkstoffen abgenommen werden.

4. Verfahren nach einem der vorhergehenden Ansprüche, **dadurch gekennzeichnet, dass** eine Kalibrierung mit Standardgasproben durchgeführt wird.

5. Verfahren nach einem der vorhergehenden Ansprüche, **dadurch gekennzeichnet, dass** bei dem Erhitzen frei gesetzte Gase unmittelbar ionenmobilitätsspektrometrisch erfasst werden bzw. vorherig in eine gaschromatografische Säule (6) eingeleitet werden.

6. Verfahren nach einem der vorhergehenden Ansprüche, **dadurch gekennzeichnet, dass** die ionenmobilitätsspektrometrische Untersuchung der Partikel mit zumindest einer Steuerung (8) für eine Produktionsstraße (1) für Holzwerkstoffe verschaltet ist.

7. Vorrichtung zum Messen von emittierenden flüchtigen Stoffen aus Holzwerkstoffen, insbesondere von Formaldehyd, vorzugsweise zur Durchführung des Verfahrens nach einem der Ansprüche 1 bis 6,
**dadurch gekennzeichnet,**
dass sie zumindest eine Einrichtung zur Probenentnahme von Partikeln (5) der Holzwerkstoffe, wenigstens eine Anlage für eine (FAIMS)-Ionenmobilitätsspektrometrie sowie eine mit Derivatisierungsmitteln versehene Anreicherungseinheit, eine thermische Desorptionseinheit und eine gaschromatografische Säule aufweist.

8. Vorrichtung nach Anspruch 7, **dadurch gekennzeichnet, dass** die Einrichtung zur Probenentnahme einer Abführleitung (12) für Säge- und/oder andere Späne der Holzwerkstoffe zugeordnet ist, wobei wenigstens ein in die Abführleitung (12) eingesetztes Ausschleusungselement (15) für die Späne vorgesehen ist.

9. Vorrichtung nach Anspruch 8, **dadurch gekennzeichnet, dass** in eine vom Ausschleusungselement (15) zur Anlage für die Ionenmobilitätsspektrometrie verlaufende Abzweigungsleitung (18) für die Späne ein Zyklonbehälter (19) und in Späneführrichtung nachfolgend eine Zellenradschleuse (20) eingesetzt sind.

10. Vorrichtung nach einem der Ansprüche 7 bis 9, **dadurch gekennzeichnet, dass** die FAIMS-Anlage (7) zumindest ein Mültikanalhochfeldionenfilter-Spektrometer bevorzugt im Druckbereich von 50 mbar bis 5000 mbar ist.

11. Vorrichtung nach einem der Ansprüche 7 bis 10, **dadurch gekennzeichnet, dass** die Anlage zur Ionenmobilitätsspektrometrie mit zumindest einer Steuerung (8) für eine Produktionsstraße (1) für Holzwerkstoffe verschaltet ist.

**Claims**

1. A method for measuring volatile organic compound emissions from wood-based materials, in particular formaldehyde, wherein a number of particles (5) are taken from a wood-based material as test specimens and investigated using ion mobility spectrometry, wherein FAIMS (7) (Field Asymmetric Waveform Ion Mobility Spectrometry) is used for the ion mobility spectrometry,
**characterized in that**
the specimens (5) are heated after they have been taken or analyzed at room temperature, gases released during heating or at room temperature are conducted through an adsorption unit (enrichment unit) with a thermal desorption unit downstream and then introduced into a gas chromatographic column (6) and that the released gases undergo derivatisation through a reaction with added chemicals, particularly in the adsorption unit.

2. The method according to claim 1, **characterized in that** it is carried out in real time during the manufacture of the wood-based material.

3. The method according to claim 1 or 2, **characterized in that** the particles (5) taken as test specimens are removed in the form of drilling chips, shavings, milling waste or sawdust, drilling cores or test samples or direct gaseous samples from wood and wood-based materials.

4. The method according to one of the preceding claims, **characterized in that** calibration is carried out using standard gas samples.

5. The method according to one of the preceding claims, **characterized in that** gases released during heating are directly detected by ion mobility spectrometry or are introduced into a gas chromatographic column (6) beforehand.

6. The method according to one of the preceding claims, **characterized in that** the investigation of the particles by ion mobility spectrometry is interconnected to at least one control unit (8) for a production line (1) for wood-based materials.

7. A device for measuring volatile organic compound emissions from wood-based materials, in particular formaldehyde, preferably for the implementation of the method according to one of claims 1 to 6,
**characterized in that**
it comprises at least one device for the sampling of particles (5) of the wood-based materials, at least one unit for ion mobility spectrometry (FAIMS) and an enrichment unit provided with derivatization means, a thermal desorption unit and a gas chromatographic column.

8. The device according to claim 7, **characterized in that** the sampling device is assigned to a removal line (12) for sawdust and/or other wood-based material chips and waste, wherein at least one discharge element (15) inserted in the removal line (12) is provided for chips and waste.

9. The device according to claim 8, **characterized in that** a cyclone container (19) for chips and waste is inserted in a branch line (18) running from the discharge element (15) to the ion mobility spectrometry unit and a rotary feeder (20) is inserted downstream in the chip and waste conducting direction.

10. The device according to one of claims 7 to 9, **characterized in that** the FAIMS unit (7) is at least a multi-channel high-field ion filter spectrometer, preferably in the pressure range from 50 mbar to 5000 mbar.

11. The device according to one of claims 7 to 10, **characterized in that** the unit for ion mobility spectrometry is interconnected to at least one control unit (8) for a production line (1) for wood-based materials.

**Revendications**

1. Procédé destiné à mesurer des matières volatiles émissives issues de dérivés du bois, notamment du formaldéhyde, lors duquel on prélève sur un dérivé du bois quelques particules (5) en tant qu'échantillons de mesure et on les analyse par spectrométrie de mobilité ionique, alors qu'on utilise pour la spectrométrie de mobilité ionique une AIMS (7) (Field Asymmetric Waveform Ion Mobility Spectrometry),
caractérisé en ce

qu'après leur prélèvement, on analyse les échantillons (5) chauffés ou à température ambiante, on fait passer les gaz libéré lors d'un chauffage ou à température ambiante à travers une unité d'adsorption (unité d'enrichissement) avec une unité de désorption thermique raccordée et on les introduit ensuite dans une colonne (6) de chromatographie gazeuse et en ce que les gaz libérés subissent notamment dans l'unité d'adsorption une dérivatisation par réaction avec des produits chimiques rajoutés.

2. Procédé selon la revendication 1, **caractérisé en ce qu'**on le réalise en temps réel, pendant la fabrication du dérivé du bois.

3. Procédé selon la revendication 1 ou la revendication 2, **caractérisé en ce qu'**on enlève les particules (5) prélevées en tant qu'échantillons de mesure sous la forme de copeaux de perçage, de rabotage, de ponçage ou de sciage, de carotte ou d'éprouvettes ou d'échantillons directement gazeux de bois ou de dérivés du bois.

4. Procédé selon l'une quelconque des revendications précédentes, **caractérisé en ce qu'**on procède à un calibrage avec des échantillons gazeux standard.

5. Procédé selon l'une quelconque des revendications précédentes, **caractérisé en ce qu'**on détecte directement par spectrométrie de mobilité ionique les gaz libérés lors du chauffage ou on les introduit préalablement dans une colonne (6) de chromatographie gazeuse.

6. Procédé selon l'une quelconque des revendications précédentes, **caractérisé en ce que** l'analyse par spectrométrie de mobilité ionique des particules est connectée avec au moins un système de commande (8) pour une ligne de production (1) de dérivés du bois.

7. Dispositif destiné à mesurer des matières volatiles émissives issues de dérivés du bois, notamment du formaldéhyde, de préférence par réalisation du procédé selon l'une quelconque des revendications 1 à 6,
**caractérisé en ce qu'**il comporte au moins un dispositif pour prélever des échantillons de particules (5) des dérivés du bois, au moins une installation pour une spectrométrie (FAIMS) de mobilité ionique, ainsi qu'une unité d'enrichissement munie de moyens de dérivatisation, une unité de désorption thermique et une colonne de chromatographie gazeuse.

8. Dispositif selon la revendication 7, **caractérisé en ce que** le dispositif pour prélever des échantillons est associé à un conduit d'évacuation (12) pour des copeaux de sciage et/ou d'autres copeaux du dérivé du bois, alors qu'il est prévu au moins un élément d'évacuation (15) des copeaux, inséré dans le conduit d'évacuation (12).

9. Dispositif selon la revendication 8, **caractérisé en ce que** dans un conduit de dérivation (18) des copeaux s'écoulant de l'élément d'évacuation (15) vers l'installation pour la spectrométrie de mobilité ionique sont insérés un récipient à cyclone (19) et dans la suite dans la direction de guidage des copeaux, une écluse à roue cellulaire (20).

10. Dispositif selon l'une quelconque des revendications 7 à 9, **caractérisé en ce que** l'installation DAIMS (7) est au moins un spectromètre multicanaux à filtre d'ion à champ d'intensité élevé, de préférence dans l'ordre de pression compris entre 50 mbar et 5000 mbar.

11. Dispositif selon l'une quelconque des revendications 7 à 10, **caractérisé en ce que** l'installation pour la spectrométrie de mobilité ionique est connectée à au moins un système de commande (8) pour une ligne de production (1) de dérivés du bois.

Fig. 1

Fig. 2

Fig. 3

EP 2 511 703 B1

Fig. 4

Proben-einlaß

Reaktions-raum

Driftraum

elektrisches Feld

Detektor

Driftgas

Verstärker

Ionisations-quelle

Einlaß-gitter

Driftringe

Abschirm-gitter

Ionenmobilitäts-Spektrum

Ionenstrom / pA

Driftzeit / ms

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2006046077 A
- US 2008054174 A
- IL 181140
- US 2008017790 A
- US 2010213364 A
- WO 2008094299 A
- WO 2009136190 A

**In der Beschreibung aufgeführte Nicht-Patentliteratur**

- **VAUTZ. W et al.** Detection of Emission from Material Surfaces using Ion Mobility Spectrometry. *INTERNATIONAL JOURNAL FOR ION MOBILITY SPECTROMETRY,* 01. Januar 2004, vol. 7 (1), 25-29
- **P. A. MARTOS ; J. PAWLISZYN.** *Anal. Chem.,* 1998, vol. 70, 2311-2320